# EUROPEAN PATENT APPLICATION

(11) **EP 2 258 208 A1**
(43) Date of publication of application: **08.12.2010**
(21) Application number: 09161730.8
(22) Date of filing: 02.06.2009
(51) Int. Cl.: A23J 1/20, A23J 3/10, A23L 1/305, A61K 8/98, A61K 38/01, A61Q 19/00, C08L 89/00

(54) **Protein Product With Modified Antigenicity**

(71) Applicant: University of Limerick, Limerick (IE)
(72) Inventor: Rajarathnam, Ebenezer, Castleroy, Limerick, (IE); O'Sullivan, Dara, Listowel, County Kerry, (IE); Fitzgerald, Dick, Limerick, (IE)
(74) Representative: McKeown, Yvonne Mary

(57) **Abstract**

A bovine casein protein hydrolysate prepared using an enzyme having broad spectrum endopeptidase activity has low residual antigenicity properties in mammals compared to intact casein protein. The composition is useful as an ingredient in foods, beverages, pharmaceutical and cosmetic products. A hydrolysate prepared using Alcalase^{™} with a degree of hydrolysis of 19.88% has very desirably low antigenicity characteristics.

SEQUENCE LISTING

<110>  UNIVERSITY OF LIMERICK

<120>  PROTEIN PRODUCT WITH MODIFIED ANTIGENICITY

<130>  P14001.EP

<160>  30

<170>  PatentIn version 3.3

<210>  1
<211>  6
<212>  PRT
<213>  Bos taurus

<400>  1

Val Leu Pro Val Pro Gln
1               5


<210>  2
<211>  6
<212>  PRT
<213>  Bos taurus

<400>  2

His Leu Pro Leu Pro Leu
1               5


<210>  3
<211>  7
<212>  PRT
<213>  Bos taurus

<400>  3

Gly Pro Phe Pro Ile Ile Val
1               5


<210>  4
<211>  8
<212>  PRT
<213>  Bos taurus

<400>  4

Ser Lys Val Leu Pro Val Pro Gln
1               5


<210>  5
<211>  10
<212>  PRT
<213>  Bos taurus

<400>  5

Ser Gln Ser Lys Val Leu Pro Val Pro Gln
1               5                   10

<210>  6
<211>  10
<212>  PRT
<213>  Bos taurus

<400>  6

Val Tyr Pro Phe Pro Gly Pro Ile Pro Asn
1               5                   10


<210>  7
<211>  12
<212>  PRT
<213>  Bos taurus

<400>  7

Thr Pro Val Val Val Pro Pro Phe Leu Gln Pro Glu
1               5                   10


<210>  8
<211>  12
<212>  PRT
<213>  Bos taurus

<400>  8

His Gln Pro His Gln Pro Leu Pro Pro Thr Val Met
1               5                   10


<210>  9
<211>  12
<212>  PRT
<213>  Bos taurus

<400>  9

Ala Val Pro Tyr Pro Gln Arg Asp Met Pro Ile Gln
1               5                   10


<210>  10
<211>  13
<212>  PRT
<213>  Bos taurus

<400>  10

Val Leu Pro Val Pro Gln Lys Ala Val Pro Tyr Pro Gln
1               5                   10


<210>  11
<211>  14
<212>  PRT
<213>  Bos taurus

<400>  11

Thr Gln Thr Pro Val Val Val Pro Pro Phe Leu Gln Pro Glu
1               5                   10

<210>  12
<211>  16
<212>  PRT
<213>  Bos taurus

<400>  12

Gln Glu Pro Val Leu Gly Pro Val Arg Gly Pro Phe Pro Ile Ile Val
1               5                   10                  15


<210>  13
<211>  14
<212>  PRT
<213>  Bos taurus

<400>  13

His Lys Glu Met Pro Phe Pro Lys Tyr Pro Val Glu Pro Phe
1               5                   10


<210>  14
<211>  16
<212>  PRT
<213>  Bos taurus

<400>  14

His Gln Pro His Gln Pro Leu Pro Pro Thr Val Met Phe Pro Pro Gln
1               5                   10                  15


<210>  15
<211>  17
<212>  PRT
<213>  Bos taurus

<400>  15

Ser Gln Ser Lys Val Leu Pro Val Pro Gln Lys Ala Val Pro Tyr Pro
1               5                   10                  15


Gln


<210>  16
<211>  17
<212>  PRT
<213>  Bos taurus

<400>  16

Met His Gln Pro His Gln Pro Leu Pro Pro Thr Val Met Phe Pro Pro
1               5                   10                  15


Gln

<210>  17
<211>  17
<212>  PRT
<213>  Bos taurus

<400>  17

Ala Pro Lys His Lys Glu Met Pro Phe Pro Lys Tyr Pro Val Glu Pro
1               5                   10                  15

Phe

<210>  18
<211>  18
<212>  PRT
<213>  Bos taurus

<400>  18

Met Ala Pro Lys His Lys Glu Met Pro Phe Pro Lys Tyr Pro Val Glu
1               5                   10                  15

Pro Phe

<210>  19
<211>  21
<212>  PRT
<213>  Bos taurus

<400>  19

Lys Phe Gln Ser Glu Glu Gln Gln Gln Thr Glu Asp Glu Leu Gln Asp
1               5                   10                  15

Lys Ile His Pro Phe
            20

<210>  20
<211>  7
<212>  PRT
<213>  Bos taurus

<400>  20

Ala Val Pro Ile Thr Pro Thr
1               5

<210>  21
<211>  15
<212>  PRT
<213>  Bos taurus

<400>  21

Gln Gly Pro Ile Val Leu Asn Pro Trp Asp Gln Val Lys Arg Asn
1               5                   10                  15

<210>  22
<211>  16
<212>  PRT
<213>  Bos taurus

<400>  22

Tyr Gln Gly Pro Ile Val Leu Asn Pro Trp Asp Gln Val Lys Arg Asn
1               5                   10                  15


<210>  23
<211>  6
<212>  PRT
<213>  Bos taurus

<400>  23

Phe Leu Pro Tyr Pro Tyr
1               5


<210>  24
<211>  9
<212>  PRT
<213>  Bos taurus

<400>  24

Ala Val Arg Ser Pro Ala Gln Ile Leu
1               5


<210>  25
<211>  9
<212>  PRT
<213>  Bos taurus

<400>  25

Val Ile Glu Ser Pro Pro Glu Ile Asn
1               5


<210>  26
<211>  8
<212>  PRT
<213>  Bos taurus

<400>  26

Asn Gln Phe Leu Pro Tyr Pro Tyr
1               5


<210>  27
<211>  9
<212>  PRT
<213>  Bos taurus

<400>  27

Ser Arg Tyr Pro Ser Tyr Gly Leu Asn
1               5

<210>  28
<211>  5
<212>  PRT
<213>  Bos taurus

<400>  28

Thr Met Pro Leu Trp
1               5

<210>  29
<211>  6
<212>  PRT
<213>  Bos taurus

<400>  29

Val Ala Pro Phe Pro Glu
1               5

<210>  30
<211>  6
<212>  PRT
<213>  Bos taurus

<400>  30

Thr Thr Met Pro Leu Trp
1               5

SEQUENCE LISTING

<110> UNIVERSITY OF LIMERICK

<120> PROTEIN PRODUCT WITH MODIFIED ANTIGENICITY

<130> P14001.EP

<160> 30

<170> PatentIn version 3.3

<210> 1
<211> 6
<212> PRT
<213> Bos taurus

<400> 1

Val Leu Pro Val Pro Gln
1               5


<210> 2
<211> 6
<212> PRT
<213> Bos taurus

<400> 2

His Leu Pro Leu Pro Leu
1               5


<210> 3
<211> 7
<212> PRT
<213> Bos taurus

<400> 3

Gly Pro Phe Pro Ile Ile Val
1               5


<210> 4
<211> 8
<212> PRT
<213> Bos taurus

<400> 4

Ser Lys Val Leu Pro Val Pro Gln
1               5


<210> 5
<211> 10
<212> PRT
<213> Bos taurus

<400> 5

Ser Gln Ser Lys Val Leu Pro Val Pro Gln
1               5                   10


<210> 6

<211>  10
<212>  PRT
<213>  Bos taurus

<400>  6

Val Tyr Pro Phe Pro Gly Pro Ile Pro Asn
1               5                   10


<210>  7
<211>  12
<212>  PRT
<213>  Bos taurus

<400>  7

Thr Pro Val Val Val Pro Pro Phe Leu Gln Pro Glu
1               5                   10


<210>  8
<211>  12
<212>  PRT
<213>  Bos taurus

<400>  8

His Gln Pro His Gln Pro Leu Pro Pro Thr Val Met
1               5                   10


<210>  9
<211>  12
<212>  PRT
<213>  Bos taurus

<400>  9

Ala Val Pro Tyr Pro Gln Arg Asp Met Pro Ile Gln
1               5                   10


<210>  10
<211>  13
<212>  PRT
<213>  Bos taurus

<400>  10

Val Leu Pro Val Pro Gln Lys Ala Val Pro Tyr Pro Gln
1               5                   10


<210>  11
<211>  14
<212>  PRT
<213>  Bos taurus

<400>  11

Thr Gln Thr Pro Val Val Val Pro Pro Phe Leu Gln Pro Glu
1               5                   10


<210>  12
<211>  16
<212>  PRT

<213>  Bos taurus

<400>  12

Gln Glu Pro Val Leu Gly Pro Val Arg Gly Pro Phe Pro Ile Ile Val
1               5                   10                  15


<210>  13
<211>  14
<212>  PRT
<213>  Bos taurus

<400>  13

His Lys Glu Met Pro Phe Pro Lys Tyr Pro Val Glu Pro Phe
1               5                   10


<210>  14
<211>  16
<212>  PRT
<213>  Bos taurus

<400>  14

His Gln Pro His Gln Pro Leu Pro Pro Thr Val Met Phe Pro Pro Gln
1               5                   10                  15


<210>  15
<211>  17
<212>  PRT
<213>  Bos taurus

<400>  15

Ser Gln Ser Lys Val Leu Pro Val Pro Gln Lys Ala Val Pro Tyr Pro
1               5                   10                  15


Gln



<210>  16
<211>  17
<212>  PRT
<213>  Bos taurus

<400>  16

Met His Gln Pro His Gln Pro Leu Pro Pro Thr Val Met Phe Pro Pro
1               5                   10                  15


Gln


<210>  17
<211>  17
<212>  PRT
<213>  Bos taurus

<400>  17

Ala Pro Lys His Lys Glu Met Pro Phe Pro Lys Tyr Pro Val Glu Pro
1               5                   10                  15

Phe

<210>  18
<211>  18
<212>  PRT
<213>  Bos taurus

<400>  18

Met Ala Pro Lys His Lys Glu Met Pro Phe Pro Lys Tyr Pro Val Glu
1               5                   10                  15

Pro Phe

<210>  19
<211>  21
<212>  PRT
<213>  Bos taurus

<400>  19

Lys Phe Gln Ser Glu Glu Gln Gln Gln Thr Glu Asp Glu Leu Gln Asp
1               5                   10                  15

Lys Ile His Pro Phe
            20

<210>  20
<211>  7
<212>  PRT
<213>  Bos taurus

<400>  20

Ala Val Pro Ile Thr Pro Thr
1               5

<210>  21
<211>  15
<212>  PRT
<213>  Bos taurus

<400>  21

Gln Gly Pro Ile Val Leu Asn Pro Trp Asp Gln Val Lys Arg Asn
1               5                   10                  15

<210>  22
<211>  16
<212>  PRT
<213>  Bos taurus

<400>  22

Tyr Gln Gly Pro Ile Val Leu Asn Pro Trp Asp Gln Val Lys Arg Asn
1               5                   10                  15

<210>  23
<211>  6
<212>  PRT
<213>  Bos taurus

<400>  23

Phe Leu Pro Tyr Pro Tyr
1               5

<210>  24
<211>  9
<212>  PRT
<213>  Bos taurus

<400>  24

Ala Val Arg Ser Pro Ala Gln Ile Leu
1               5

<210>  25
<211>  9
<212>  PRT
<213>  Bos taurus

<400>  25

Val Ile Glu Ser Pro Pro Glu Ile Asn
1               5

<210>  26
<211>  8
<212>  PRT
<213>  Bos taurus

<400>  26

Asn Gln Phe Leu Pro Tyr Pro Tyr
1               5

<210>  27
<211>  9
<212>  PRT
<213>  Bos taurus

<400>  27

Ser Arg Tyr Pro Ser Tyr Gly Leu Asn
1               5

<210>  28
<211>  5
<212>  PRT
<213>  Bos taurus

<400>  28

Thr Met Pro Leu Trp
1               5

<210>  29
<211>  6
<212>  PRT
<213>  Bos taurus

<400>  29

Val Ala Pro Phe Pro Glu
1               5

<210>  30
<211>  6
<212>  PRT
<213>  Bos taurus

<400>  30

Thr Thr Met Pro Leu Trp
1               5

## Description

The invention relates to a protein hydrolysate preparation with enhanced functional properties and to a method for its production. The invention also relates to a method for the preparation of a composition suitable for use in comestible products. In particular the invention relates to a product having reduced residual antigenicity properties.

Recent marketplace trends in comestibles, including foods and beverages, indicate a growing demand for so-called "functional foods" or food supplements. Such foods may provide the consumer with an advantage which extends beyond the purely nutritional value of the food.

Research over the last 25 years has begun to identify and focus on food and its constituents as a source of safe, biologically active (bioactive) components having or likely to have the ability positively to influence the prevention, amelioration, day-to-day consequences or progression of various human health/medical or animal health/veterinary conditions, and/or meet the health enhancement/lifestyle desires and expectations of various consumer groups. In recent years, for instance, the use of milk components as ingredients in functional foods or nutraceuticals or use as functional foods or nutraceuticals in their own right has been gaining increasing, worldwide scientific credibility. Also, increasing awareness by consumers of the link between diet and health/wellness is globally raising demand for these product categories.

One example of such food is a food composition which includes sterol or stanol esters, which provide a reduction in circulating cholesterol.

Functional foods may additionally or alternatively contain components which provide an advantageous physical property to the food or food supplement. Functional foods generally provide some real, perceived or alleged health enhancing or maintenance effect, or include some component which provides protection against disease, or which has some real, perceived or alleged health promoting effects. Other so-called functional foods may include foods enriched with additive or supplements, including live microorganisms or probiotic materials.

The newer term lifestyle foods is applied to foods, which are becoming associated by the consumer with benefits such as: general wellness, energy, avoidance or diminution of allergic reactions, alertness, weight management, physical appearance, emotional wellbeing, longevity etc. The growing consumer interest in these emerging food categories is leading to an increase in self-medication, which is being driven mainly by the desire to avoid the undesirable side-effects associated with the use of synthetic drugs and to stem the increasing cost burden associated with conventional drug therapies.

As used herein, the word "food" is intended to include solid, semi-solid and liquid consumables intended for ingestion into the human or animal body.

Certain additives may be added to edible and potable consumable foods which provide enhanced technical properties, such as improved heat stability, better foaming or antifoaming characteristics, altered solubility properties, modified organoleptic features or many other variables.

Such alterations in the properties include the preparation of materials which are less likely to induce an allergic reaction or response in individuals who are susceptible to such reactions. Materials which provide a reduced risk of provoking an allergic response are often referred to as having "reduced antigenicity" or in some cases as "hypoallergenic". Where a material is to be used in animals, particularly in mammals and most especially in humans, whether administered topically, parenterally, orally or by any other route, it is preferable that the material should have reduced antigenicity or be non-allergenic or hypoallergenic. This is particularly important for individuals who are at higher risk of displaying allergic response, such as the young, the elderly and immune-compromised individuals.

As used herein, the term "reduced antigenicity" refers to a modified material which elicits a lesser immune response or no immune response compared to the unmodified material from which it is derived. Reduced antigenicity in the context of the present invention indicates the characteristics of a material/modified material which yields a reduction in antibody binding/response as a consequence of enzymatic degradation/hydrolysis of antigenic epitopes/sequences in the primary sequences of the caseins. As used herein, the term "hypoallergenic" refers to protein hydrolysates which have been modified (enzymatically, in this instance) to significantly reduce or even abolish their activity as mediators of an allergic reaction in a subject, particularly a susceptible subject, challenged with the material.

Casein is the main protein constituent of milk and is an abundant source of protein or protein-product for use in materials, including but not limited to foods, food supplements, cosmetics, pharmaceutical products and the like in which a source of nitrogen, amino acids, peptides and/or proteins is desirable.

The present invention relates to a composition for inclusion as an ingredient in formulations which provide a source of nitrogen, amino acids, peptides and/or proteins and which has reduced antigenicity, non-allergenic or hypoallergenic characteristics.

An object of the present invention is therefore to provide a composition derived from casein having modified antigenicity properties.

Accordingly, the present invention provides a composition comprising a milk protein hydrolysate prepared by treating milk casein protein with a proteolytic enzyme having broad-specificity endopeptidase activity, the composition having reduced antigenicity in mammals compared to intact milk casein protein. Preferably, the enzyme activity comprises subtilisin or subtilisin-like activity and/or glutamyl endopeptidase or glutamyl endopeptidase-like activity.

In a preferred embodiment, the proteolytic enzyme is derived from *Bacillus* species, in particular from *Bacillus licheniformis* and in a most preferred arrangement the proteolytic enzyme comprises Alcalase^{™} from *Bacillus licheniformis.*

We have found that a casein protein hydrolysate prepared with the Alcalase^{™} from *Bacillus licheniformis* and having a degree of hydrolysis of greater than about 3%, preferably from about 10% to about 20% has a log reduction in antigenicity of between about 2.7 to about 5 fold compared to intact casein.

Ideally, the degree of hydrolysis is in the range of from about 3% to about 20%, preferably in excess of about 11% and more preferably about 13% or about 20% and most preferably about 19.88%.

The composition of the invention ideally includes a casein hydrolysate which has greater than 80% solubility between pH 6.0 to 8.0.

In one embodiment, the composition includes one or more peptides? having the sequence of any of SEQ.ID No. 1 to SEQ.ID No. 30.

In an important aspect, the composition of the invention has application for use as an ingredient with reduced residual antigenicity. It also has use as a "hypoallergenic" ingredient which elicits little or no (detectable) allergic response.

The reduced residual antigenicity or hypoallergenic casein hydrolysates of the invention have use as ingredients in the food, cosmetics and pharmaceutical industries. In the food industry, the composition has utility in various food and drink products, included but not limited to yoghurts and yoghurt drinks and desserts, including energy foods, sports beverages, and supplements. In particular, it is very useful in food nutrients for individuals who require special feeding regimes, such as elderly people who are unable to consume normal food stuffs, persons on special diets and the like. It may also be useful for those who display, or are at higher risk of displaying, allergic reactions to milk proteins, particularly, the casein proteins. This is a matter of known relevance to formulations for feeding infants.

The composition according to the invention prepared using Alcalase^{™} with a degree of hydrolysis of about 19.88% has special application in consumables requiring reduced antigenicity/hypoallergenic ingredients as described above. Such consumables include infant foods, foods for immune-compromised patients and foods for individuals who display allergenic reactions to the consumption of milk proteins, specifically the caseins.

In the cosmetics and pharmaceutical industries, the compositions of the invention are very useful in products for topical use.

The proteolytic enzyme is preferably derived from *Bacillus* species, most preferably from *Bacillus licheniformis.* In a preferred selection, the proteolytic enzyme comprises Alcalase^{™} from *Bacillus licheniformis.*

Ideally, the hydrolysate has no peptides with a molecular mass of greater than 10 kDa.

In another aspect, the invention provides a composition having reduced antigenicity for use as an ingredient in foods, beverages, cosmetics and/or pharmaceuticals.

The invention will now be described more particularly with reference to the accompanying drawings, in which:
Figure 1 is a table showing the molecular mass distribution profile of the Alcalase™ hydrolysates of casein;
Figure 2 shows the nitrogen solubility profiles as a function of degree of hydrolysis (DH %) for the Alcalase™ hydrolysates of casein;
Figure 3 shows the heat stability properties of the casein hydrolysates generated using Alcalase™;
Figure 4 is a table which shows the residual antigenicity of the Alcalase™ hydrolysates of casein; and
Figure 5 is a table showing the sequences of some peptides present in an 19.88 % DH Alcalase ™ hydrolysate of casein

The present invention relates to milk protein hydrolysates which display desirable characteristics. In particular, we have found that a hydrolysate prepared from milk casein has good reduced antigencity compared to intact milk casein protein. Casein protein is presently considered as a poor choice for formulated foods for management of the nutritional requirements of certain human subjects who are at higher risk of suffering allergic responses than most of the population. Infants in particular often display an allergenic reaction to intact casein. The present invention provides a modified casein protein ingredient which has desirable characteristics of reduced residual antigenicity compared to intact casein protein.

In the description below, we refer in particular to casein protein as sodium caseinate derived from bovine milk, but it is to be understood that other casein/caseinate protein sources are equally useful in the present invention.

### Generation of hydrolysates:

Sodium caseinate (NaCN, 85.92% w/w protein) was purchased from a commercial supplier of bovine milk in Ireland. NaCN hydrolysates were generated using Alcalase™, a food grade proteolytic preparation derived from *Bacillus licheniformis.* Salt caseinates other than sodium caseinates may equally be used in the preparation of casein protein from milk for use in the present invention and the invention is to be understood to extend to caseinates generally. For hydrolysis, aqueous solutions of NaCN ranging in concentration from 9.3 (w/v) protein were incubated at 50 °C at pH 7. The pH was maintained constant using a pH stat (Titrino 718, Metrohm, Herisau, Switzerland) charged with 2 mol L⁻¹ NaOH as previously described (Flanagan & FitzGerald, 2002). Enzyme inactivation was by heat treatment at 80 °C for 20 min. Hydrolysate samples were stored at -18 °C prior to subsequent analysis. The protein content in the NaCN substrate was determined using the macro-Kjeldahl method (IDF, 1993) and a Kjeldahl conversion factor of 6.38 was used.

The reaction conditions used to generate the hydrolysates are outlined in Table 1 below.

**Table 1: Hydrolysate manufacturing conditions.**

| **Protein substrate** | **Proteolytic Preparation** | **Experimental conditions** |
|---|---|---|
| Sodium caseinate (9.3 % w/v) | Alcalase^{™} 2.4L (0.23 mL per 100 mL of protein solution) (Nova Nordisk A/S) | pH 7, 50 °C |

### Quantification of hydrolysate functional properties

### Nitrogen solubility index determination

This was carried out essentially according to Flanagan and FitzGerald (2002)

Nitrogen was determined on the supernatants of 4 g 100 g⁻¹ aqueous protein solutions following centrifugation (1700 x g; 30 min), using a modification of the macro-Kjeldahl method (IDF, 1993). Kjeldahl catalyst tablets were used instead of potassium and copper sulphate, and the end-point of the titration step was reached at pH 4.6. All nitrogen solubility analyses were carried out in duplicate.

### Molecular mass distribution profiles

Gel permeation HPLC (GP-HPLC) was performed using the Waters HPLC system as previously described(Spellman *et al.* (2005). Hydrolysate samples were diluted to 0.2 % (w/v) in H₂O, filtered through 0.2 µm syringe filters and 20 µl applied to a TSK G2000 SW separating column (600 × 7.5 mm ID) connected to a TSKGEL SW guard column (75 × 7.5 mm ID). Separation was by isocratic elution with a mobile phase of 0.1 % TFA in 30 % acetonitrile, at a flow rate of 0.5 ml min⁻¹. Detector response was monitored at 214 nm. A calibration curve was prepared from the average retention times of standard proteins and peptides (Smyth & FitzGerald, 1998). The void volume (Vₒ) was estimated with thyroglobulin (600 000 Da) and the total column volume (Vₜ) was estimated with L-tyrosine.HCl (218 Da).

### Heat stability

This was performed essentially as described by Ryan *et al.* (2008)

For heat stability studies, the pH of aliquots of aqueous NaCN hydrolysates (2.0% (w/v) protein equivalent) were adjusted to between 2.0 and 8.0, using 0.1 M and 1 M HCl or NaOH. Samples were then allowed to equilibrate for at least 1 h at room temperature. The pH of the samples was then re-measured and re-adjusted, if necessary, prior to heat stability analysis. Immediately after final pH adjustment, samples (2 ml) were placed in glass tubes (10 mm i.d. _ 120 mm, AGB Scientific, Dublin, Ireland), sealed with silicone bungs, immersed in an oil bath thermostatically controlled at 140°C (Elbanton BV, Kerkdriel,The Netherlands), with continuous rocking at motor speed setting 3. The heat coagulation time (HCT) was taken as the length of time in minutes that elapsed between placing the sample in the oil bath and the onset of coagulation (McSweeney, Mulvihill, & O'Callaghan, 2004). These analyses were performed in triplicate.

### Residual antigenicity

This was quantified using an enzyme linked immunoassay (ELISA) protocol employing polyclonal antisera raised in rabbit against unhydrolysed sodium caseinate.

Rabbits were immunised with a representative casein immunogen preparation. Aliquots of the NaCN hydrolysate samples were analysed for residual antigenicity to intact casein using the polyclonal antisera raised in rabbits. Plots of ELISA response (A₄₅₀/₆₀₀) versus log protein concentration were created to determine the relative reduction in residual antigenicity to intact casein (expresses as log reduction).

### Liquid chromatography (LC) and mass spectroscopy (MS) characterisation of hydrolysates

The casein hydrolysate samples were analyzed by LC-MS using an UltiMate® 3000 Nano LC instrument (Dionex, Sunnyvale, USA) coupled to a MicrOTOF II mass spectrometer (Bruker Daltonics, Bremen, Germany) equipped with an electrospray ion source. Test sample (1 µL) was first loaded onto a C₁₈ PepMap 100 Precolumn Cartridge (Dionex, Sunnyvale, USA) at a flow rate of 25 µL min⁻¹ for 3min. Sample was eluted from the trap column and separated on a C18 PepMap 100 column (Dionex, 75 µm × 150mm, 3 µm, Sunnyvale, USA) at a flow rate of 3 50 nL min⁻¹ with a mobile phase containing water and 0.1 % (v/v) formic acid (mobile phase A) and acetonitrile containing 0.1 % (v/v) formic acid (mobile phase B) employing a linear gradient from 4.0 to 40% B in 35 min. The column was thermostated at 25°C. The MS and tandem MS experiments were controlled by the MicrOTOF control software (version 2.3, Bruker Daltonics). Data were acquired over a mass/charge (m/z) range of 300-2500. A full scan MS spectrum was acquired, followed by tandem mass spectra using collision-induced dissociation (CID) of the five most intense precursor ions present in the MS scan. Electrospray conditions were as follows: capillary temperature, 130°C; Capillary voltage, -1500V; Dry Gas flow, 6.0 L min⁻¹. DataAnalysis software (version 4.0, Bruker Daltonics), BioTool (version 3.1, Bruker Daltonics) and MASCOT (David N. Perkins, 1999) were used for data processing and evaluation of peptide sequence.

### Results

### Molecular Mass Distribution Profiles

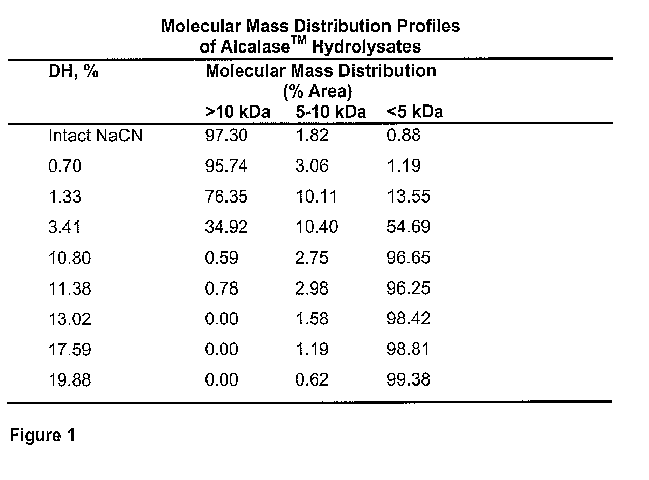

The results in Figure 1 summarise the molecular mass distribution profiles of the hydrolysates as a function of degree of hydrolysis (DH). This Figure also indicates the terminal DH value achieved during hydrolysis with Alcalase™. The terminal DH value achieved and percentage of peptide material > 10 kDa for the Alcalase™ hydrolysate were 19.88% DH and 0.00 > 10 kDa, respectively. Such terminal DH % values and molecular mass distribution profiles are parameters which are specified by end-users dependent on the ultimate ingredient application for which the hydrolysate is intended.

In many instances high levels of low molecular mass peptides with minimal levels of large molecular mass components (> 10 kDa) is desirable in hydrolysate samples particularly for application in reduced antigenicity/hypoallergenic food systems.

### Nitrogen solubility index

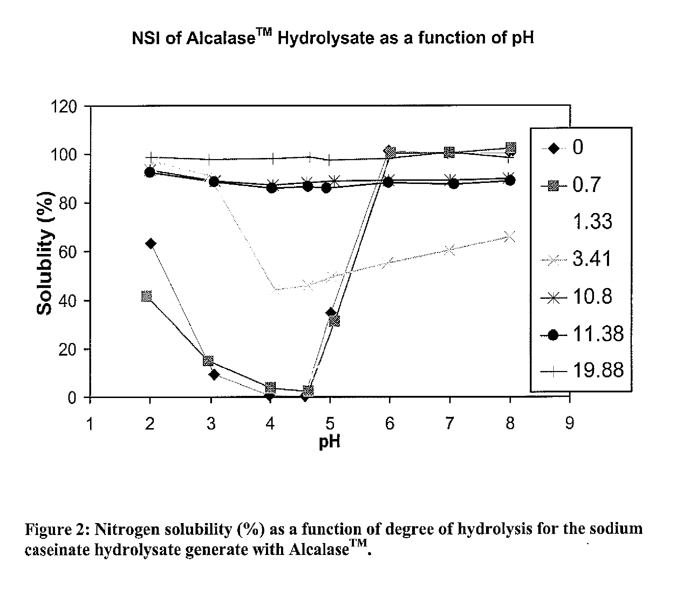

It is evident from the results (Figure 2) that as the degree of hydrolysis increases the solubility of the hydrolysates also increases across the pH range tested. It is also evident that variable changes in solubility occur for low DH samples which are pH dependent. Good nitrogen solubility is generally a prerequisite for the utilisation of food proteins and their hydrolysates as functional and biofunctional ingredients in formulated food systems.

### Heat stability

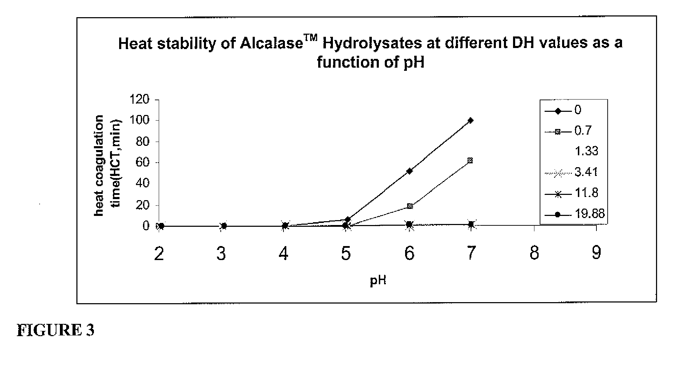

The results from heat stability analyses at 140°C are shown in Figure 3. At high pH (pH 6.0 and 7.0), the hydrolysates at low DH had good heat stability properties. This, coupled with the associated reduction in residual antigenicity described below, improved solubility and enhanced molecular mass distribution profiles is a favourable functional characteristic in the utilisation of these low DH hydrolysates in neutral pH formulated foods which may be subjected to high heat treatments during processing.

### Residual antigenicity

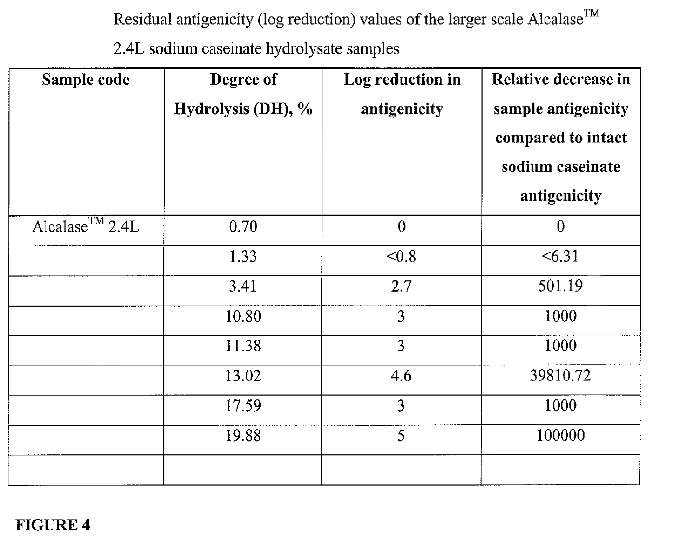

The results for the residual antigenicity of various Alcalase™ hydrolysates having differing degrees of hydrolysis (DH) values are shown in Figure 4. The Alcalase™ hydrolysate sample had a highly significant (10⁵ fold) reduction in residual antigenicity in comparison to intact NaCN. Low levels of residual antigenicity are favourable in terms of utilising protein hydrolysates as ingredients in reduced allergenicity foods specifically in the case of humans susceptible to allergenic reactions to intact caseins.

The 19.88 % DH Alcalase^{™} NaCN hydrolysate having a 10⁵ log reduction in residual antigenicity is within the range required by infant formulae manufacturers for the production of hypoallergenic product. As shown in Figure 1, this hydrolysate has no high molecular mass material (> 10 kDa) a property linked to its acceptability as a reduced antigenicity/hypoallergenic ingredient.

### Peptide composition in Alcalase™ hydrolysates of sodium caseinate

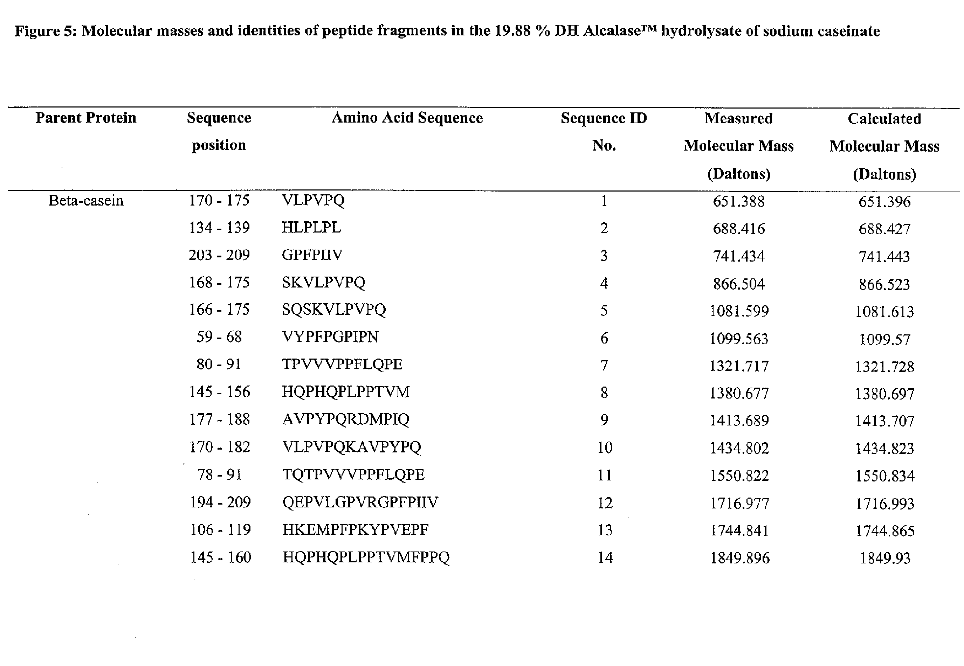

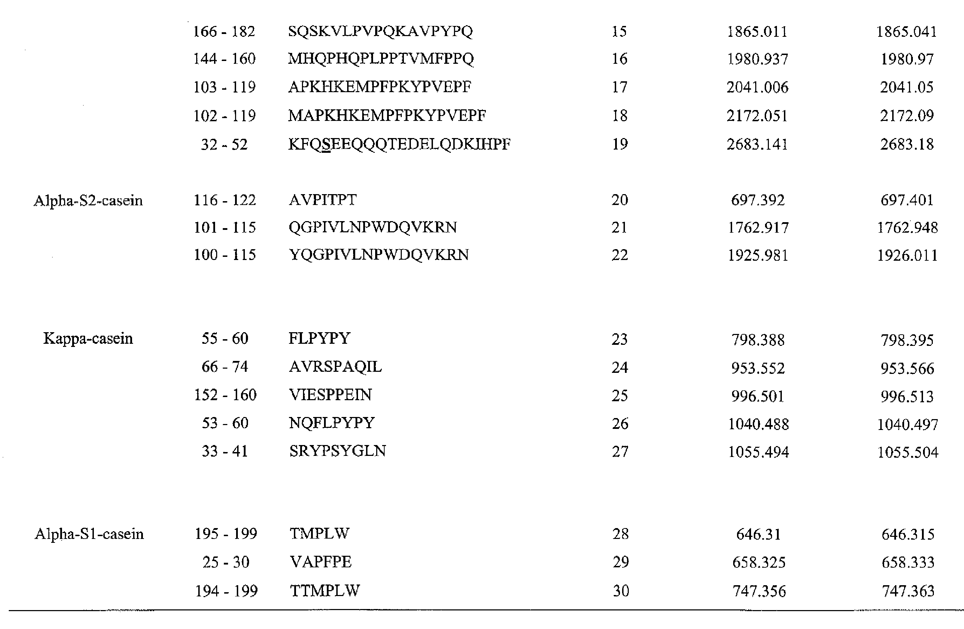

Figure 5 shows molecular masses and identities of peptide fragments in the 19.88% Alcalase^{™} sodium caseinate hydrolysate. As described above, peptides in the mass/charge (m/z) range of 300 to 2500 were characterised by LC-MS. The sequences of the peptides of the four bovine casein proteins alpha-S1, alpha-S2, beta and kappa have been described and we were thus able to cross-reference the peptides of the 19.88% hydrolysate with the native protein as set out in Figure 5.

The full sequences of the individual caseins are summarised in:
'Chemistry of the Caseins' Chapter 3 by H. E. Swaisgood in: Advanced Dairy Chemistry Vol 1 Proteins Third Ed Part A (eds Fox and McSweeney), Kluwer Academic/ Plenum Publishers.

The typical total number of residues for each individual casein protein are as follows:
αₛ₁ - casein 199 residues (variant B)
αₛ₂ - casein 207 residues (variant A)
β -casein 209 residues (variant B)
κ-casein 169 residues (variant A²)

The specific genetic variants are given in brackets above for those variants typically present in bovine milk from Western breeds.

It is evident that peptide fragment originating from the four caseins ( αₛ₁, αₛ₂, β- and κ-) are present in the hydrolysates and that most of the peptides result from endopeptidase activity.

The sequence listing which follows shows the amino acid sequences of the 30 peptides we found in the m/z range of 300 to 2500 of the Alcalase^{™} 19.88% DH preparation.

### References

Flanagan, J. and FitzGerald, R. J. (2002). Physicochemical and nitrogen solubility properties of Bacillus proteinase hydrolysates of sodium caseinate incubated with transglutaminase pre- and post-hydrolysis. J. Agric. Food Chem. 50(19): 5429-5436.
IDF 20B (1993). *Milk: Determination of Nitrogen Content. Kjeldahl Method.* Brussels, Belgium: International Dairy Federation.
McSweeney, S.L., Mulvihill, D.M., & O' Callaghan, D.M. (2004). The influence of pH on the heat-induced aggregation of model milk protein ingredient systems and model infant formula emulsions stabilized by milk protein ingredients, Food Hydrocolloids, 18 (1), 109-125.
Perkins, D. N., Creasy, D. M. and Cottrell, J. S. (1999). Probability-based protein identification by searching sequence databases using mass spectrometry data. Electrophoresis, 20, 3551-3567.
Ryan, M., McEvoy, E., Duignan, S., Crowley, C., Fenelon, M., O' Callaghan, D.M. and FitzGerald, R.J. (2008). Thermal stability of soy protein isolate and hydrolysate ingredients. Food Chem. 108:503-510.
Smyth, M. and FitzGerald, R. J. (1998). Relationship between some characteristics of WPC hydrolysates and the enzyme complement in commercially available proteinase preparations. Int. Dairy J. 8: 819-827.
Spellman, D. O'Cuinn, G. and FitzGerald, R. J. (2005) Physicochemical and sensory characteristics of whey protein hydrolysates generated at different total solids levels. J. Dairy Res. 72: 138-143.

It is to be understood that the invention is not limited to the specific details described herein which are given by way of example only and that various modifications and alterations are possible without departing from the scope of the invention as defined by the appended claims.

## Claims

1. A composition comprising a milk casein protein hydrolysate having peptides generated by treating milk casein protein with a proteolytic enzyme having broad-specificity endopeptidase activity, the composition having reduced antigenicity in mammals compared to intact milk casein protein.

2. The composition of claim 1 wherein the enzyme activity used to generate the peptides comprises subtilisin or subtilisin-like activity and/or glutamyl endopeptidase or glutamyl endopeptidase-like activity.

3. The composition of claim 2, wherein the proteolytic enzyme is derived from *Bacillus* species.

4. The composition of claim 3, wherein the proteolyte enzyme is derived from *Bacillus licheniformis.*

5. The composition of any of claims 1 to 4, wherein the proteolytic enzyme comprises Alealase^{™} from *Bacillus licheniformis.*

6. The composition of claim 5, wherein the casein protein has a degree of hydrolysis of greater than about 3%.

7. The composition of claim 6, wherein the casein protein has a degree of hydrolysis of about 10% to about 20%.

8. The composition of any of claims 5 to 7, wherein the degree of hydrolysis is in excess of about 11% and more preferably about 13% or about 20%.

9. The composition of any of claims 5 to 8, wherein the casein protein has a degree of hydrolysis of about 19.88% and has no peptides with a molecular mass of greater than 10 kDa.

10. The composition of any of claims 1 to 9, wherein the composition has a log reduction in antigenicity of between about 2.7 to about 5 fold compared to intact casein.

11. The composition of any of claims 1 to 10, wherein the casein hydrolysate has greater than 80% solubility between pH 2.0 to 8.0.

12. The composition of any of claims 1 to 11, including one or more peptide having the sequence of any of SEQ.ID No. 1 to SEQ.ID No. 30.

13. Use of a composition as claimed in any of claims 1 to 12 as an ingredient with reduced residual antigenicity compared to intact casein protein.

14. A composition as claimed in any of claims 1 to 12 for use as an ingredient in a food, beverage, pharmaceutical or cosmetic product.

15. A composition as claimed in claim 14 for use as an ingredient in food and beverages for infants, children, elderly persons and immune-compromised patients.
